(19) Europäisches Patentamt
European Patent Office
Office européen des brevets

(11) **EP 2 347 708 A1**

(12) **EUROPEAN PATENT APPLICATION**
published in accordance with Art. 153(4) EPC

(43) Date of publication:
**27.07.2011 Bulletin 2011/30**

(51) Int Cl.:
***A61B 5/1455*** (2006.01)   ***A61B 5/026*** (2006.01)

(21) Application number: **09820627.9**

(22) Date of filing: **16.10.2009**

(86) International application number:
**PCT/JP2009/067873**

(87) International publication number:
**WO 2010/044449 (22.04.2010 Gazette 2010/16)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **17.10.2008 JP 2008269101**

(71) Applicant: **Sysmex Corporation**
**Kobe-shi, Hyogo 651-0073 (JP)**

(72) Inventor: **OZAWA, Toshiyuki**
**Kobe-shi**
**Hyogo 651-0073 (JP)**

(74) Representative: **HOFFMANN EITLE**
**Patent- und Rechtsanwälte**
**Arabellastraße 4**
**81925 München (DE)**

(54) **MONITORING DEVICE FOR LIVING BODY**

(57) This living body monitoring apparatus includes image acquisition means emitting light to a living body and acquiring a living body image by imaging the living body to which the light is being emitted, concentration acquisition means acquiring a concentration of a blood component of the living body by analyzing the living body image obtained by the image acquisition means, information acquisition means acquiring information about a change of a circulating blood volume contained in the living body on the basis of the concentration of the blood component obtained by the concentration acquisition means, and output means outputting the information about a change of a circulating blood volume obtained by the information acquisition means.

FIG.6

**Description**

Technical Field

The present invention relates to a living body monitoring apparatus for monitoring changes of a circulating blood volume in a living body.

Background Art

As a technique of measuring changes of a circulating blood volume, a technique of measuring a circulating blood volume in addition to hemodialysis is known in general, as described in Patent Document 1, for example.

Prior Art Document

Patent Document

Patent Document 1: Japanese National Patent Publication Gazette No. 2001-502590

Summary of the Invention

Problems to be Solved by the Invention

However, a well-known technique of measuring a circulating blood volume is invasive, as described in the aforementioned Patent Document 1, and a burden on a patient is large. A burden, on a patient susceptible to a vasovagal reaction (VVR) is especially large.
The present invention has been made in view of the situation as described above, and an object of the present invention is to provide a living body monitoring apparatus capable of monitoring changes of a circulating blood volume noninvasively.

Means for Solving the Problems and Effect of the Invention

In order to solve the aforementioned problems, a living body monitoring apparatus according to a first aspect of the present invention comprises image acquisition means emitting light to a living body and acquiring a living body image by imaging the living body to which the light is being emitted, concentration acquisition means acquiring a concentration of a blood component of the living body by analyzing the living body image obtained by the image acquisition means, information acquisition means acquiring information about a change of a circulating blood volume contained in the living body on the basis of the concentration of the blood component obtained by the concentration acquisition means, and output means outputting the information about a change of a circulating blood volume obtained by the information acquisition means.
In this living body monitoring apparatus according to the first aspect, as hereinabove described, the concentration acquisition means acquires the concentration of the blood component of the living body by analyzing the living body image obtained by the image acquisition means, the information acquisition means acquires the information about a change of a circulating blood volume contained in the living body and the output means outputs the information about a change of a circulating blood volume obtained by the information acquisition means, whereby the change of the circulating blood volume can be noninvasively monitored.
Preferably in the aforementioned living body monitoring apparatus according to the first aspect, the information acquisition means is configured to acquire the information about a change of a circulating blood volume on the basis of a first concentration of the blood component obtained by analyzing a first living body image obtained by the image acquisition means and a second concentration of the blood component obtained by analyzing a second living body image obtained after acquisition of the first living body image. According to this structure, the information about a change of a circulating blood volume can be acquired on the basis of the concentrations of the blood component at two points, which are the first concentration of the blood component and the second concentration of the blood component.
Preferably in this case, the living body monitoring apparatus further comprises standard circulating blood volume acquisition means acquiring a standard circulating blood volume capable of being calculated from a height and a weight of the living body, wherein the information acquisition means acquires the information about a change of a circulating blood volume on the basis of the first concentration of the blood component, the second concentration of the blood component and the standard circulating blood volume. According to this structure, the information about a change of a circulating blood volume can be acquired on the basis of a circulating blood volume normally contained in the living body.

Preferably in the aforementioned structure further comprising the standard circulating blood volume acquisition means, the information acquisition means acquires a circulating blood volume in the living body at the time of acquisition of the second living body image by obtaining a change rate of the concentration of the blood component on the basis of the first concentration of the blood component and the second concentration of the blood component and accumulating the change rate of the concentration of the blood component and the standard circulating blood volume, and the output means outputs the standard circulating blood volume and the circulating blood volume in the living body at the time of acquisition of the second living body image as the information about a change of a circulating blood volume. According to this structure, a circulating blood volume corresponding to the magnitude of the change (change rate) of the concentration of the blood component can be acquired, and hence a user can see the change of the circulating blood volume at a glance by outputting the circulating blood volume corresponding to the change of the concentration of the blood component and the standard circulating blood volume.

Preferably in the aforementioned living body monitoring apparatus according to the first aspect, the blood component is hemoglobin. According to this structure, the accurate information about a change of a circulating blood volume on the basis of a correlation between a change of a concentration of hemoglobin and the change of the circulating blood volume can be acquired.

Preferably in the aforementioned structure in which the information acquisition means acquires the information about a change of a circulating blood volume on the basis of the first concentration of the blood component and the second concentration of the blood component, the information acquisition means is configured to be capable of acquiring the information about a change of a circulating blood volume on the basis of the first concentration of the blood component obtained by analyzing the first living body image, the second concentration of the blood component obtained by analyzing the second living body image, and a third concentration of the blood component obtained by analyzing a third living body image obtained after acquisition of the second living body image. According to this structure, the information about a change of a circulating blood volume is acquired with the third concentration of the blood component in addition to the first concentration of the blood component and the second concentration of the blood component, whereby the more detailed information about a change of a circulating blood volume can be output.

Preferably in the aforementioned living body monitoring apparatus according to the first aspect, the output means is configured to output a graph showing a relation between time lapse and the circulating blood volume contained in the living body as the information about a change of a circulating blood volume. According to this structure, information (a graph) with which the user easily visually understands the change of the circulating blood volume can be output.

Preferably, the aforementioned structure further comprising the standard circulating blood volume acquisition means further comprises input acceptance means accepting input of body information about a height and a weight of a living body, wherein the standard circulating blood volume acquisition means acquires the standard circulating blood volume on the basis of the body information accepted by the input acceptance means. According to this structure, the standard circulating blood volume can be easily acquired with the body information accepted by the input acceptance means.

Preferably in this case, the input acceptance means is further configured to be capable of accepting input of a volume of collected blood collected from the living body, and the standard circulating blood volume acquisition means is configured to be capable of acquiring the standard circulating blood volume on the basis of the body information and the volume of collected blood accepted by the input acceptance means. According to this structure, the standard circulating blood volume can be acquired on the basis of the volume of collected blood accepted by the input acceptance means in addition to the body information, and hence the standard circulating blood volume can be accurately acquired also when collecting blood.

A living body monitoring apparatus according to a second aspect of the present invention comprises an image acquisition portion emitting light to a living body and acquiring a living body image by imaging the living body to which the light is being emitted, an output portion, and a control portion acquiring a concentration of a blood component of the living body by analyzing the living body image obtained by imaging the living body by the image acquisition portion, acquiring information about a change of a circulating blood volume contained in the living body on the basis of the obtained concentration of the blood component, and outputting the obtained information about a change of a circulating blood volume to the output portion.

As hereinabove described, this living body monitoring apparatus according to the second aspect comprises the image acquisition portion acquiring the concentration of the blood component of the living body by analyzing the living body image obtained by imaging the living body and the control portion acquiring the information about a change of a circulating blood volume contained in the living body on the basis of the obtained concentration of the blood component and outputting the obtained information about a change of a circulating blood volume to the output portion, whereby the change of the circulating blood volume can be noninvasively monitored.

Preferably in the aforementioned living body monitoring apparatus according to the second aspect, the control portion is configured to acquire a first concentration of the blood component by analyzing a first living body image obtained by the image acquisition portion, acquire a second concentration of the blood component by analyzing a second living body image obtained after acquisition of the first living body image, and acquire the information about a change of a circulating blood volume on the basis of the first concentration of the blood component and the second concentration of the blood component. According to this structure, the information about a change of a circulating blood volume can be acquired on the basis of the concentrations of the blood component at two points, which are the first concentration of the blood component and the second concentration of the blood component.

Preferably in this case, the control portion is configured to acquire a standard circulating blood volume on the basis of information of a height and a weight of the living body, and acquire the information about a change of a circulating blood volume on the basis of the first concentration of the blood component, the second concentration of the blood component and "the standard circulating blood volume. According to this structure, the information about a change of a circulating blood volume can be acquired on the basis of a circulating blood volume normally contained in the living body.

Preferably in the aforementioned structure in which the control portion acquires the standard circulating blood volume, the control portion is configured to obtain a change rate of the concentration of the blood component on the basis of the first concentration of the blood component and the second concentration of the blood component, acquire a circulating blood volume in the living body at the time of acquisition of the second living body image from a product of the change rate and the standard circulating blood volume, and output the standard circulating blood volume and the circulating blood volume in the living body at the time of acquisition of the second living body image to the output portion as the information about a change of a circulating blood volume. According to this structure, a circulating blood volume corresponding to the magnitude of the change (change rate) of the concentration of the blood component can be acquired, and hence a user can see the change of the circulating blood volume at a glance by outputting the circulating blood volume corresponding to the change of the concentration of the blood component and the standard circulating blood volume.

Preferably in the aforementioned living body monitoring apparatus according to the second aspect, the blood component is hemoglobin. According to this structure, the accurate information about a change of a circulating blood volume on the basis of a correlation between a change of a concentration of hemoglobin and the change of the circulating blood volume can be acquired.

Preferably in the aforementioned structure in which the control portion acquires the information about a change of a circulating blood volume on the basis of the first concentration of the blood component and the second concentration of the blood component, the control portion is configured to acquire the information about a change of a circulating blood volume on the basis of the first concentration of the blood component obtained by analyzing the first living body image, the second concentration of the blood component obtained by analyzing the second living body image, and a third concentration of the blood component obtained by analyzing a third living body image obtained after acquisition of the second living body image. According to this structure, the information about a change of a circulating blood volume is acquired with the third concentration of the blood component in addition to the first concentration of the blood component and the second concentration of the blood component, whereby the more detailed information about a change of a circulating blood volume can be output.

Preferably in the aforementioned living body monitoring apparatus according to the second aspect, the control portion is configured to output a graph showing a relation between time lapse and the circulating blood volume contained in the living body to the output portion as the information about a change of a circulating blood volume. According to this structure, information (a graph) with which the user easily visually understands the change of the circulating blood volume can be output.

Preferably in the aforementioned structure in which the control portion acquires the standard circulating blood volume, the control portion is configured to accept input of body information about a height and a weight of the living body, and acquire the standard circulating blood volume on the basis of the accepted body information. According to this structure, the standard circulating blood volume can be easily acquired with the body information accepted by the control portion.

Preferably in this case, the control portion is configured to accept input of a volume of collected blood collected from the living body, and acquire the standard circulating blood volume on the basis of the accepted body information and the volume of collected blood. According to this structure, the standard circulating blood volume can be acquired on the basis of the volume of collected blood accepted by the control portion in addition to the body information, and hence the standard circulating blood volume can be accurately acquired also when collecting blood.

Preferably in the aforementioned living body monitoring apparatus according to the second aspect, the control portion includes a first control portion acquiring the concentration of the blood component of the living body by analyzing the living body image obtained by imaging the living body by the image acquisition portion, and a second control portion acquiring the information about a change of a circulating blood volume contained in the living body on the basis of the concentration of the blood component obtained by the first control portion and outputting the obtained information about a change of a circulating blood volume to the output portion. According to this structure, in a case where the first control portion controlling the image acquisition portion acquires the concentration of the blood component and transmits the concentration of the blood component to the second control portion, for example, it is not necessary to transmit image data having a large amount of data but data of the concentration of the blood component (numeric data) having a smaller amount of data than the image data can be simply transmitted, and hence data processing can be efficiently performed.

Brief Description of the Drawings

[Fig. 1] A diagram schematically showing the structure of a monitoring apparatus according to an embodiment of the present invention.
[Fig. 2] A block diagram schematically showing the structure of a PC of the monitoring apparatus according to the embodiment shown in Fig. 1.
[Fig. 3] A block diagram schematically showing the structure of a detecting portion of the monitoring apparatus according to the embodiment shown in Fig. 1.
[Fig. 4] A flow chart showing an operation of the monitoring apparatus according to the embodiment shown in Fig. 1.
[Fig. 5] A diagram showing an example of a standby screen displayed on a display portion of the PC of the monitoring apparatus according to the embodiment shown in Fig. 1.
[fig. 6] A diagram showing an example of a measurement screen displayed on the display portion of the PC of the monitoring apparatus according to the embodiment shown in Fig. 1.
[Fig. 7] A flow chart showing a subroutine of data analysis by the monitoring apparatus according to the embodiment shown in Fig. 4.
[Fig. 8] A flow chart showing a subroutine of hemoglobin concentration measurement by the monitoring apparatus according to the embodiment shown in Fig. 4.
[Fig. 9] A diagram showing an example of a luminance profile acquired in the hemoglobin concentration measurement by the monitoring apparatus according to the embodiment shown in Fig. 8.
[Fig. 10] A diagram showing an example of a concentration profile acquired in the hemoglobin concentration measurement by the monitoring apparatus according to the embodiment shown in Fig. 8.
[Fig. 11] A diagram graphically illustrating changes of hemoglobin concentration in an experimental example of the monitoring apparatus according to the embodiment shown in Fig. 1.
[Fig. 12] A diagram graphically illustrating changes of circulating blood volume in the experimental example of the monitoring apparatus according to the embodiment shown in Fig. 1.

Modes for Carrying Out the Invention

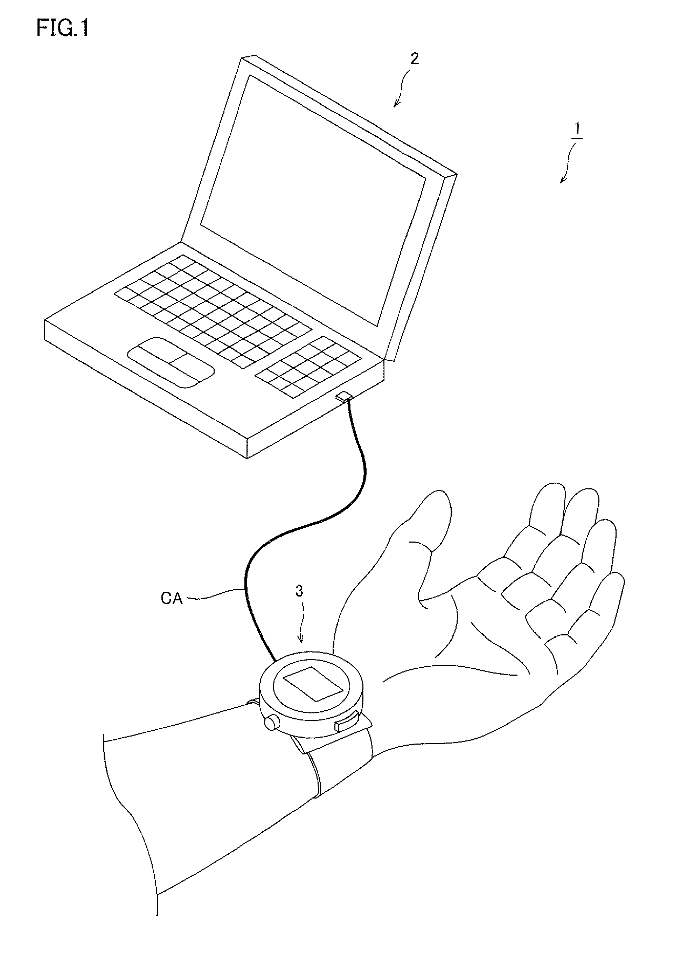

An embodiment of the present invention is hereinafter described on the basis of the drawings. First, the structure of a monitoring apparatus 1 according to the embodiment of the present invention is described in detail with reference to Figs. 1 to 3.

As shown in Fig. 1, the monitoring apparatus 1 according to this embodiment is employed by mounting a detecting portion 3 on the wrist of a subject from whom blood has been collected or is going to be collected. More specifically, the monitoring apparatus 1 is mainly employed to monitor how the circulating blood volume of the subject having been reduced by the collection of blood has changed by oral fluid intake of the subject for preventing the risk of a VVR for the subject.

A VVR mean that vagus nerves become tense by collection of blood thereby causing a person from whom blood is collected blood pressure reduction or bradycardia. Blood pressure reduction or bradycardia reduces oxygen supply to the brain, and hence the person from whom blood is collected is also at risk of falling down after the collection of blood due to a VVR. It is known that the risk of blood pressure reduction or bradycardia due to a VVR is reduced by fluid replacement. In other words, whereas the circulating blood volume is rapidly reduced by the collection of blood, the reduced circulating blood volume is recovered by absorbing fluid as a plasma component, and consequently blood pressure reduction or bradycardia can be minimized. Therefore, in the monitoring apparatus 1 according to this embodiment, the change of the circulating blood volume due to fluid replacement can be noninvasively measured while a secondary VVR is prevented.

The monitoring apparatus 1 comprises the detecting portion 3 emitting light to a blood vessel existing in the subject's wrist and measuring a hemoglobin (Hb) concentration on the basis of a living body image obtained by imaging the blood vessel to which the light has been emitted and a personal computer (PC) 2 calculating a blood dilution rate and a circulating blood volume by analyzing the Hb concentration obtained by the detecting portion 3 and outputting the calculated blood dilution rate and circulating blood volume. The detesting portion 3 and the PC 2 are connected with each other through a connecting cable CA. The specific structures of the PC 2 and the detecting portion 3 are hereinafter described in detail.

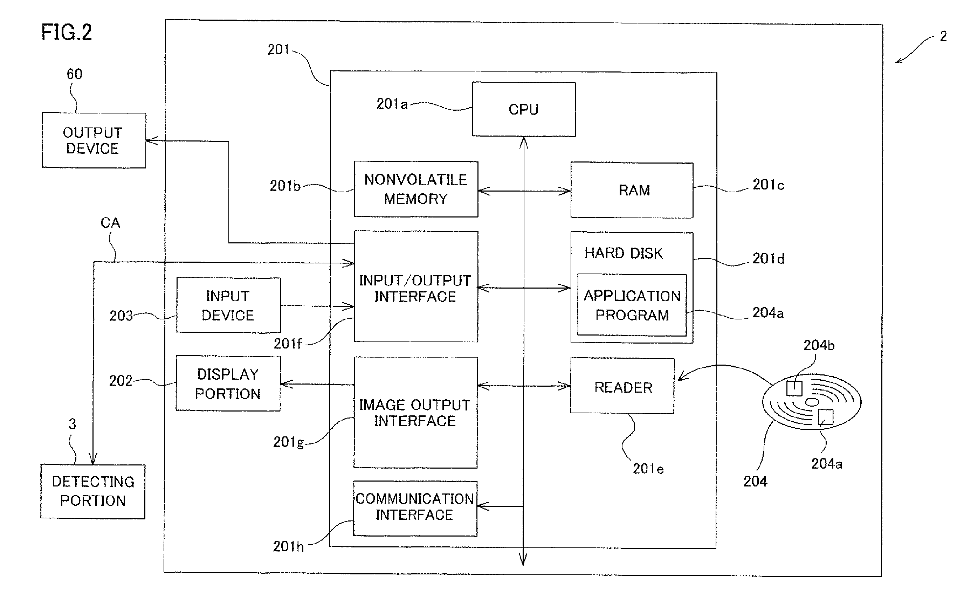

As shown in Fig. 2, the PC 2 includes a control portion 201, a display portion 202 and an input device 203. The control portion 201 is constituted by a CPU 201a, a nonvolatile memory 201b, a RAM 201c, a hard disk 201d, a reader 201e, an input/output interface 201f, an image output interface 201g and a communication interface 201h. The CPU 201a, the nonvolatile memory 201b, the RAM 201c, the hard disk 201d, the reader 201e, the input/output interface 201f, the image output interface 201g and the communication interface 201h are connected with each other by a bus.

The CPU 201a is provided for running computer programs stored in the nonvolatile memory 201b and computer programs read by the RAM 201c. Further, the CPU 201a has functions of acquiring analysis data by processing results of the measurement of the Hb concentration obtained by the detecting portion 3 and outputting an image signal corresponding to a screen for displaying the analysis data to the image output interface 201g.

The nonvolatile memory 201b is constituted by a rewritable nonvolatile memory such as a flash memory. The computer programs run by the CPU 201a, data employed for these, and the like are recorded in this nonvolatile memory 201b. The RAM 201c is constituted by an SRAM, a DRAM or the like. The RAM 201c is employed for reading the computer programs recorded in the nonvolatile memory 201b and the hard disk 201d. Further, the RAM 201c is utilized as a working area of the CPU 201a when running these computer programs.

An operating system and various computer programs such as application programs to be run by the CPU 201a as well as data employed for running the computer programs are installed in the hard disk 201d. An application program 204a described later is also installed in this hard disk 201d.

The reader 201e is constituted by a flexible disk drive, a CD-ROM drive, a DVD-ROM drive or the like, and can read computer programs or data recorded in a portable recording medium 204 or the like. The portable recording medium 204 stores the application program 204a for allowing the computer to realize a prescribed function and an install program 204b for installing the application program 204a in the computer. When the install program 204b is run, the computer serving as the PC 2 can read the application program 204a from the portable recording medium 204 and install the application program 204a in the hard disk 201d.

Further, the operating system such as Windows (registered trademark) manufactured and sold by Microsoft, U.S.A., for example, providing graphical user interface environment is installed in the hard disk 201d. In the following description, it is assumed that the application program 204a according to this embodiment operates on the aforementioned operating system.

The input/output interface 201f is constituted by a serial interface such as USB, IEEE 1394 or RS-232C, a parallel interface such as SCSI, IDE or IEEE 1284, an analog interface configured by a D/A converter, an A/D converter and so on, or the like, for example. The input/output interface 201f is connected with the input device 203 constituted by a keyboard and a mouse. A user can input data into the PC 2 by using the input device 203. Further, the input/output interface 201f is connected with an output device 206 constituted by a printer etc. Further, the input/output interface 201f is connected with the detecting portion 3 by the connecting cable CA (see Fig. 1). Thus, the PC 2 is configured to be capable of transmitting data to and receiving data from the detecting portion 3 through the input/output interface 201f.

The communication interface 201h is Ethernet (registered trademark) interface. The PC 2 is configured to be capable of performing communication with an external network by the communication interface 201h with a prescribed communication protocol (TCP/IP).

The image output interface 201g is connected to the display portion 202 and configured to output an image signal supplied from the CPU 201a to the display portion 202. The display portion 202 is configured to display an image (screen) according to the input image signal.

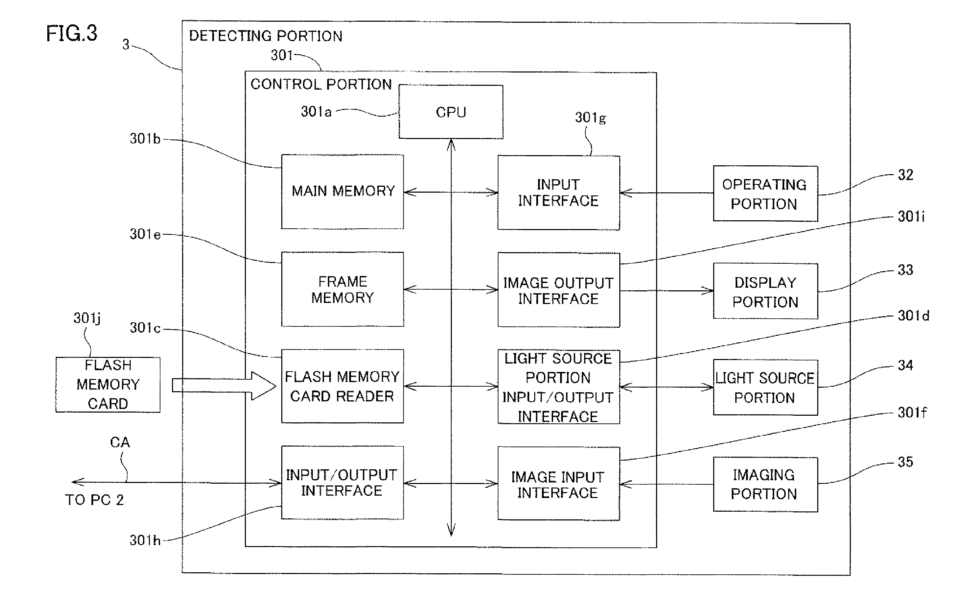

The detecting portion 3 is a wristwatch-type portable measuring device (see Fig. 1). As shown in Fig. 3, the detecting portion 3 comprises a control portion 301, an operating portion 32, a display portion 33, a light source portion 34 and an imaging portion 35.

The control portion 301 controls an operation of each portion of the detecting portion 3. The operating portion 32 comprises a plurality of keys provided on a body of the detecting portion 3 and is configured to accepting an operation by the user. The display portion 33 comprises a liquid crystal panel provided on an upper surface of the body of the detecting portion 3 and is configured to display information for guiding an operation, error information, etc. The light source portion 34 and the imaging portion 35 are provided on a bottom surface of the body of the detecting portion 3. The light source portion 34 comprises a light-emitting diode for emitting near-infrared light (center wavelength of 830 nm) of a wavelength having a high absorption rate by hemoglobin and is configured to emit light to the wrist of the subject on which the detecting portion 3 is mounted. The imaging portion 35 comprises a CCD camera and is configured to acquire a living body image by imaging the wrist to which the light has been emitted by the light source portion 34.

The control portion 301 comprises a CPU 301a, a main memory 301b, a flash memory card reader 301c, a light source portion input/output interface 301d, a frame memory 301e, an image input interface 301f, an input interface 301g, an input/output interface 301h and an image output interface 301i. The CPU 301a, the main memory 301b, the flash memory card reader 301c, the light source portion input/output interface 301d, the frame memory 301e, the image input interface 301f, the input interface 301g, the input/output interface 301h and the image output interface 301i are connected with each other through a data transmission line so as to be capable of transmitting data mutually. Due to this structure, the CPU 301a can read and write data corresponding to each of the main memory 301b, the flash memory card reader 301c and the frame memory 301e, and transmit and receive data corresponding to each of the light source portion input/output interface 301d, the image input interface 301f, the input interface 3C1g, the image output interface 301i and the input/output interface 301h.

The CPU 301a is configured to be capable of running a computer program read by the main memory 301b. The main memory 301b is constituted by an SRAM, a DRAM or the like. The main memory 301b is employed for reading computer programs stored in a flash memory card 301j. Further, the main memory 301b is utilized as a working area of the CPU 301a when running these computer programs.

The flash memory card reader 301c is employed for reading data stored in the flash memory card 301j. The flash memory card 301j has a flash memory (not shown) and is configured to be capable of holding data without supplying power from outside. Further, the flash memory card 301j stores computer programs run by the CPU 301a, data employed in running the computer programs and so on.

An operating system such as the TRON specification-compliant operating system, for example, is installed on the flash memory card 301j. Note that the operating system is not restricted to this, but an operating system such as Windows (registered trademark) manufactured and sold by Microsoft, U.S.A., for example, providing graphical user interface environment may be employed.

The light source portion input/output interface 301d is constituted by an analog interface configured by a D/A converter, an A/D converter and so on. The light source portion input/output interface 301d is electrically connected with the light-emitting diode (not shown) provided in the light source portion 34 by an electrical signal line. The light source portion input/output interface 301d is configured to control an operation of the light-emitting diode of the light source portion 34.

The frame memory 301e is constituted by an SRAM, a DRAM or the like. The frame memory 301e is utilized for storing data when the image input interface 301f described later executes image processing. The image input interface 301f comprises a video digitize circuit (not shown) including an A/D converter. The image input interface 301f is electrically connected to the imaging portion 35 by an electrical signal line, so that an image signal is input from the imaging portion 35 to the image input interface 301f. The image signal input from the imagine portion 35 is A/D converted by the image input interface 301f. Image data digital converted as described above is stored in the frame memory 301e.

The input interface 301g is constituted by an analog interface configured by an A/D converter. The input interface 301g is electrically connected with the operating portion 32. Due to this structure, the user can turn on/off the power of the apparatus, initialize the apparatus and so on through the operating portion 32.

The input/output interface 301h is constituted by a serial interface such as USB, IEEE 1394 or RS-232C or a parallel interface such as SCSI, for example. The control portion 301 is configured to be capable of transmitting data to and receiving data from the PC 2 through the connecting cable CA by this input/output interface 301h.

The image output interface 301i is electrically connected to the display portion 33 and configured to output an image signal based on image data supplied from the CPU 301a to the display portion 33.

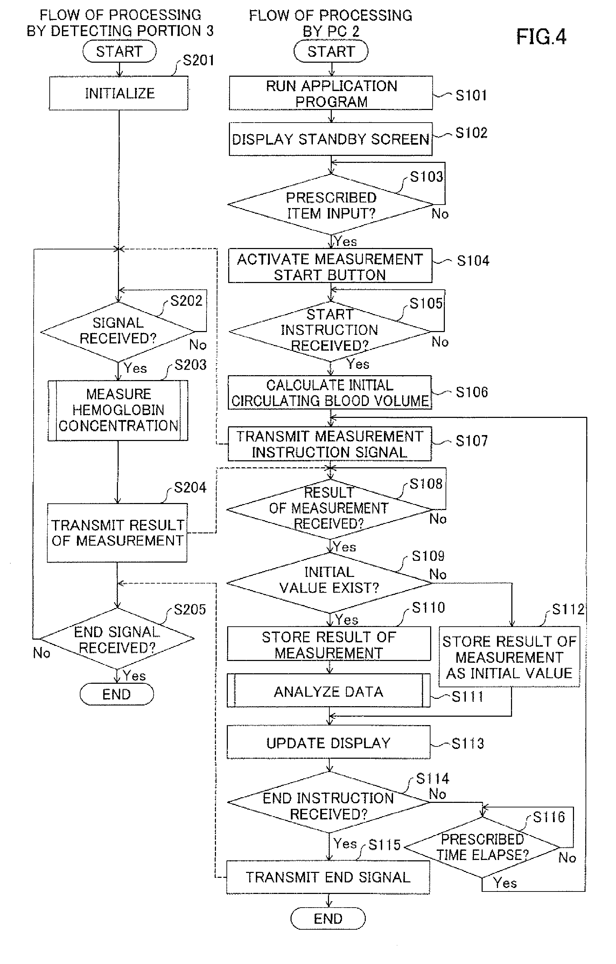

Next, an operation of the monitoring apparatus 1 according to this embodiment is described with reference to Figs. 4 to 10.

As hereinabove described, the monitoring apparatus 1 according to this embodiment is employed to monitor how the circulating blood volume of the subject having been reduced by collection of blood has changed by oral fluid intake of the subject. Therefore, a person from whom blood has been collected before the start of measurement by the monitoring apparatus 1 according to this embodiment or a person from whom blood is going to be collected after the start of measurement, who has taken fluid orally before and after the collection of blood (in 10 minutes before and after the collection of blood, for example), becomes an object person of the monitoring apparatus 1. In the following description, a person from whom blood has been collected before the start of measurement by the monitoring apparatus 1 and taken fluid orally before the start of measurement is measured.

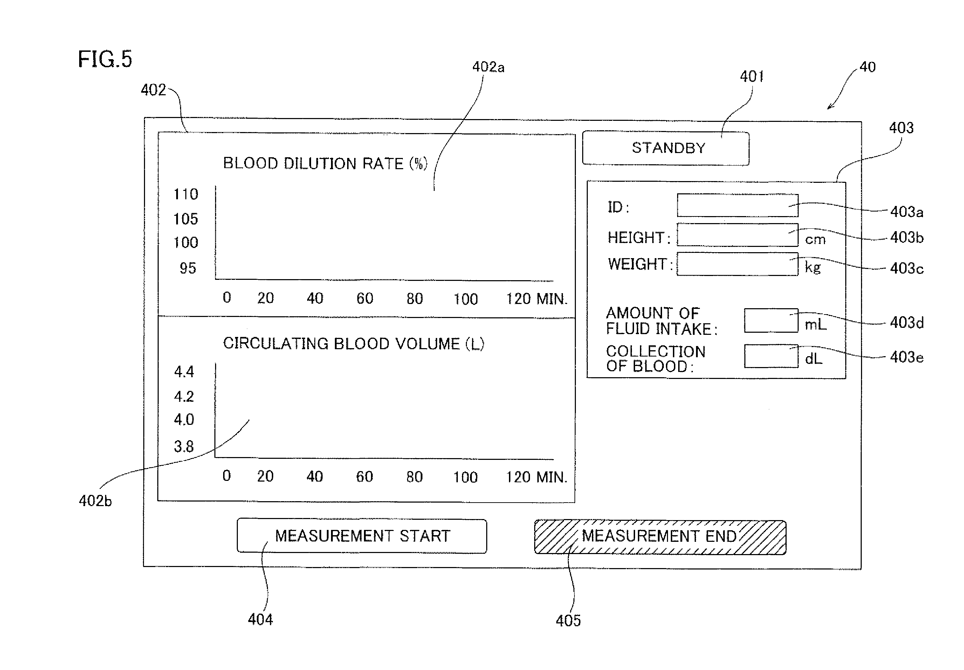

As shown in Fig. 4, at a step S101, processing is started when a user operates the PC 2 thereby running an application program for executing a function of the monitoring apparatus 1. When the application program is run, the CPU 201a executes processing for displaying a standby screen 40 (see Fig. 5) on the display portion 202 of the PC 2 at a step S102.

As shown in Fig. 5, the standby screen 40 displayed on the display portion 202 at the step S102 includes a status display portion 401, a graph display portion 402, a subject data display portion 403, a measurement start button 404 and a measurement end button 405.

On the status display portion 401, whether the monitoring apparatus 1 is performing measurement or is in a standby state is displayed. In a state shown in Fig. 5, measurement has not been started, and hence standby" is displayed.

The graph display portion 402 includes a first display region 402a and a second display region 402b. Blood dilution rates calculated by algorithm described later are displayed as a graph on the first display region 402a. Circulating blood volumes calculated by algorithm described later are displayed as a graph on the second display region 402b. In the state shown in Fig. 5, measurement has not been started, and hence no graph has been displayed on the first display region 402a and the second display region 402b.

On the subject data display portion 403, subject data constituted by the ID, the height, the weight, the amount of fluid intake and the volume of collected blood of the subject is displayed with respect to each item. The subject data display portion 403 is so configured that the subject data can be input on a screen, and input boxes 403a to 403e corresponding to each item are displayed. The user can specify a desired input box with a pointing device (input device 203) such as a mouse and input each item by operating a keyboard (input device 203). In a case where 200 dL of blood is collected from the subject and the subject takes 200 mL of fluid orally after the collection of blood and before the start of measurement, for example, the user inputs "200 (mL)" into the input box 403d corresponding to the amount of fluid intake and "200 (dL)" into the input box 403e corresponding to the volume of collected blood.

The measurement start button 404 is so provided that the user can instruct the monitoring apparatus 1 to start measurement. The measurement start button 404 is activated (acceptance of input is enabled) by input into the input boxes 403a to 403e corresponding to prescribed items displayed on the subject data display portion 403. The measurement end button 405 is so provided that the user can instruct the monitoring apparatus 1 to end measurement after the measurement is started. The measurement, end button 405 is activated by the start of measurement.

When displaying the standby screen 40 at the step S102, the CPU 201a determines whether or not the prescribed items have been input into the respective input boxes 403a to 403e of the subject data display portion 403 of the standby screen 40 at a step S103. When determining that the prescribed items have not been input, the CPU 201a returns the process to the step S103.

When the user inputs the prescribed items of the subject data display portion 403, the CPU 201a activates the measurement start button 404 displayed on the standby screen 40 (see Fig. 5) at a step S104. At a step S105, the CPU 201a determines whether or not a measurement instruction from the user has been accepted through the measurement start button 404 . When determining that the measurement instruction has been accepted, the CPU 201a advances the process to a step S106. When determining that the measurement instruction has not been accepted, on the other hand, the CPU 201a returns the process to the step S105.

Next, at the step S106, the CPU 201a executes processing for calculating an initial circulating blood volume. More specifically, the CPU 201a calculates the initial circulating blood volume of the subject on the basis of values of the subject's height (h) and weight (w) input on the standby screen 40. The initial circulating blood volume is obtained on the basis of the following formula (1):

$$\text{initial circulating blood volume (L)} = 0.168 \times (h/100) \times 3 + (0.05 \times w + 0.444) \; ... \; (1)$$

The formula (1), which is a formula for obtaining a circulating blood volume from a height and a weight, is well-known as the formula of Fujita and Ogawa, and the detailed description is omitted.

At this time, the CPU 201a determines whether or not a volume of collected blood has been input into the input box 403e of the subject data display portion 403 of the standby screen 40 (see Fig. 5). When the volume of collected blood has been input, the CPU 201a subtracts the volume of collected blood input into the input box 403e from the initial circulating blood volume obtained by the aforementioned formula (1) and sets the obtained value to an initial circulating blood volume. Then, the CPU 201a stores the obtained initial circulating blood volume in the nonvolatile memory 201b.

Next, at a step S107, the CPU 201a executes processing for transmitting a measurement instruction signal to the detecting portion 3.

Then, at a step S108, the CPU 201a determines whether or not a result of measurement of Hb concentration has been received from the detecting portion 3. When determining that the result of measurement has been received, the CPU 201a advances the process to a step S109. When determining that the result of measurement has not been received, on the other hand, the CPU 201a returns the process to the step 5108.

At the step S109, the CPU 201a determines whether or not an initial value of the Hb concentration has been stored in the nonvolatile memory 201b. The term "initial value" here is a result of measurement of Hb concentration initially transmitted from the detecting portion 3 after the start of measurement. When determining that the initial value has been stored, namely, when the detecting portion 3 performs second or later Hb concentration measurement after the start of measurement, the CPU 201a advances the process to a step S110. On the other hand, when determining that the initial value has not been stored, namely, when the detecting portion 3 performs first Hb concentration measurement after the start of measurement, the CPU 201a advances the process to a step S112.

When the process advances to the step S112, the CPU 201a stores the result of measurement transmitted from the detecting portion 3 as the initial value in the nonvolatile memory 201b.

When the process advances to the step S110, the CPU 201a executes processing for storing the result of measurement transmitted from the detecting portion 3 in the nonvolatile memory 201b. At a step S111, the CPU 201a performs data analysis according to a prescribed routine. The CPU 201a calculates a blood dilution rate and a circulating blood volume reflecting a latest result of measurement of Hb concentration through this data analyzing processing. The data analysis is described in detail later.

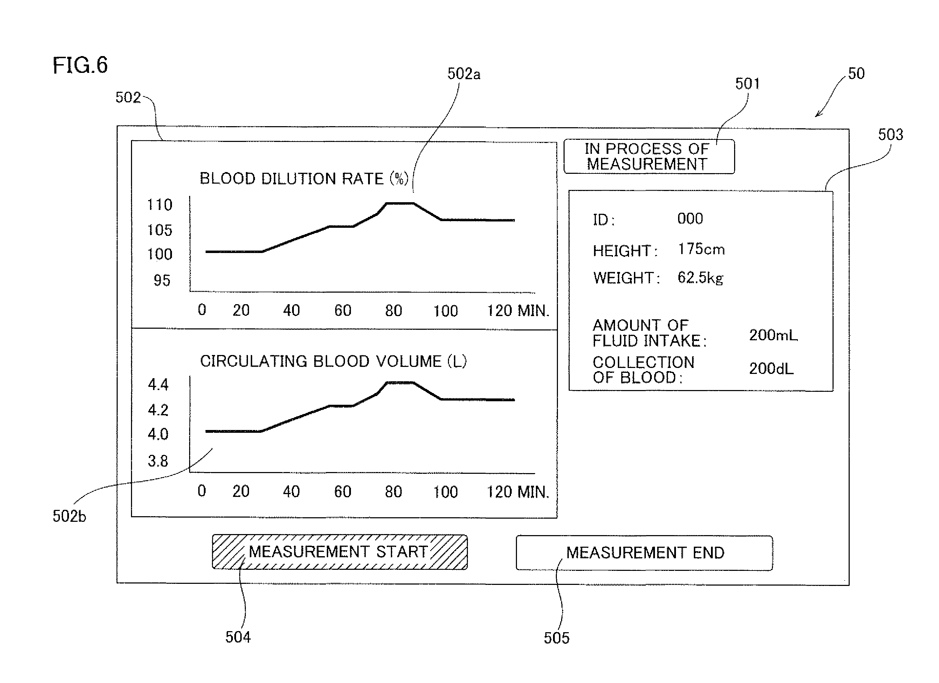

At a step S113, the CPU 201a executes processing for updating display of the display portion 202 of the PC 2. As shown in Fig. 6, a measurement screen 50 is a screen displayed on the display portion 202 of the PC 2 in measurement. The measurement screen 50 includes a status display portion 501, a graph display portion 502, a subject data display portion 503, a measurement start button 504 and a measurement end button 505, similarly to the aforementioned standby screen 40.

As shown in Fig. 6, a message saying that the monitoring apparatus 1 is measuring is displayed on the status display portion 501. On a first display region 502a. of the graph display portion 502, a graph (blood dilution rate graph) showing a fluctuation of a blood dilution rate is displayed. On a second display region 502b of the graph display portion 502, a graph (circulating blood volume graph) showing a fluctuation of a circulating blood volume is displayed. On the subject data display portion 503, the subject data input on the standby screen 40 is displayed.

At this step S113, the CPU 201a executes processing for updating display of the graphs displayed on the graph display portion 502. In other words, the CPU 201a generates a blood dilution rate graph including a latest blood dilution rate on the basis of the blood dilution rate obtained through the data analyzing processing at the step S111. Further, the CPU 201a generates a circulating blood volume graph showing a fluctuation of a circulating blood volume over time on the basis of an initial circulating blood volume and a latest blood dilution rate. The CPU 201a executes processing for displaying the generated blood dilution rate graph and circulating blood volume graph on the first display region 502a and second display region 502b, respectively. Thus, a graph reflecting an updated result of measurement is displayed on the measurement screen 50 of the display portion 202 every time the detecting portion 3 measures an Hb concentration. The user can know how much the circulating blood volume having been reduced by the collection of blood before the start of measurement has been recovered by oral fluid intake by referring to the circulating blood volume graph displayed on the measurement screen 50.

Thereafter, at a step S114, the CPU 201a determines whether or not an instruction to end measurement has been accepted from the user. More specifically, the CPU 201a determines whether or not the instruction to end measurement from the user has been accepted through the measurement end button 505 displayed on the measurement screen 50. When determining that the instruction to end measurement has not been accepted, the CPU 201a advances the process to a step S116. When determining that the instruction to end measurement has been accepted, on the other hand, the CPU 201a advances the process to a step S115.

When determining that the instruction to end measurement has not been accepted, the CPU 201a determines whether or not a prescribed time has elapsed at the step S116. The prescribed time is 90 seconds from the time when the latest result of measurement of Hb concentration stored in the nonvolatile memory 201b has been acquired. When determining that the prescribed time has not elapsed, the CPU 201a repeats the similar processing until the prescribed time elapses. When determining that the prescribed time (90 seconds) has elapsed, on the other hand, the CPU 201a returns the process to the step S107 again. Thus, every time the prescribed time elapsed, the processing at the steps S107 to S114 and S116 is automatically executed, and the display (measurement screen 50) of the display portion 202 is updated in real time. This processing is repeated until the CPU 201a determines that the instruction to end measurement has been accepted at the step S114.

When determining that the instruction to end measurement has been accepted at the step S114, the CPU 201a executes processing for transmitting a measurement end signal to the detecting portion 3 at the step S115, and the process in the PC 2 is ended. Thus, the process by the PC 2 ends.

Next, a flow of processing by the detecting portion 3 is described.

When the detecting portion 3 is powered on, the CPU 301a of the detecting portion 3 performs initialization of each part including the main memory 301b and the frame memory 301e at a step S201. Then, the CPU 301a determines whether or not the measurement instruction signal has been received from the PC 2 at a step S202. When the measurement instruction signal has been transmitted from the PC 2 at the step S107 and the measurement instruction signal has been received in the detecting portion 3, the CPU 301a advances the process to a step S202. When the measurement instruction signal has not been received from the PC 2, on the other hand, the CPU 3 returns the process to the step S202.

Thereafter, the CPU 301 a executes processing for measuring an Hb concentration of the subject according to a prescribed routine at a step S203. An Hb concentration measurement routine is described in detail later.

Next, at a step S204, the CPU 301a executes processing for transmitting the result of measurement of the Hb concentration obtained at the step S203 to the PC 2.

Thereafter, the CPU 301a determines whether or not the measurement end signal from the PC 2 has been received at a step S205. When determining that the measurement end signal has not been received from the PC 2, the CPU 301a returns the process to the step S202. As hereinabove described, the PC 2 is configured to transmit a measurement instruction signal every time the prescribed time (90 seconds) elapses (see the steps S107 to S114 and S116), and hence the detecting portion 3 is controlled to execute the processing at the steps S203 and S204 every time the prescribed time elapses. When the CPU 301a determines that the measurement end signal has been received at the step S205, the detecting portion 3 ends the process.

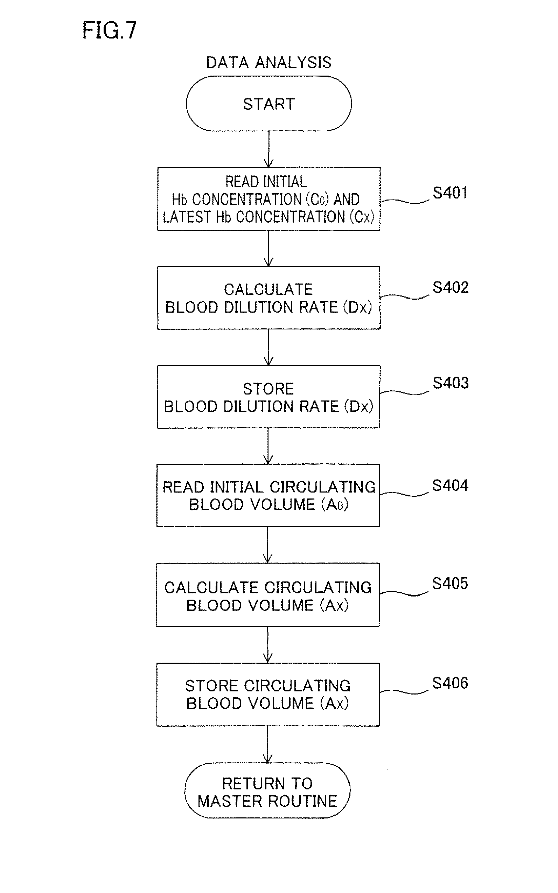

Next, a subroutine of data analysis by the PC 2 at the step S111 in Fig. 4 is described with reference to Fig. 7. In the following description, an Hb concentration is notated as C, a blood dilution rate is notated as D and a circulating blood volume is notated as A, and the respective values at a certain point x are notated as $C_x$, $D_x$ and $A_x$ while the respective initial values are notated as $C_0$, $D_0$ and $A_0$.

As shown in Fig. 7, the CPU 201a of the PC 2 reads an initial value ($C_0$) of Hb concentration and a latest Hb concentration ($C_x$) stored in the nonvolatile memory 201b at a step S401. Next, the CPU 201a calculates a blood dilution rate ($D_x$ with the read Hb concentration ($C_0$)and Hb concentration ($C_x$) at a step S402. The blood dilution rate ($D_x$) is obtained by the following formula (2) :

$$\text{blood dilution rate } (D_x) = C_0/C_x \times 100 \; (\%) \; \dots \; (2)$$

Thus, the blood dilution rate ($D_x$) is expressed by a ratio of the Hb concentration ($C_0$) to the Hb concentration ($C_x$.) and indicates a change rate of Hb concentration at the certain point x.

After the blood dilution rate ($D_x$) is calculated, the CPU 201a stores the obtained blood dilution rate ($D_x$) in the nonvolatile memory 201b at a step S403.

Next, the CPU 201a reads an initial circulating blood volume ($A_0$) stored in the nonvolatile memory 201b at a step S404. At a step S405, a circulating blood volume ($A_x$) at the certain point x is calculated with the blood dilution rate ($D_x$) and the initial circulating blood volume ($A_0$) obtained at the aforementioned step S402. The circulating blood volume ($A_x$) is obtained by the following formula (3):

$$\text{volume } (A_0) \times \text{blood dilution rate } (D_x)/100 \; \dots \; (3)$$

Thus, the circulating blood volume ($A_x$) at the certain point x is calculated by accumulating the blood dilution rate ($D_x$) which is a change rate of hemoglobin. concentration at the certain point x and the initial circulating blood Volume ($A_0$).

Thereafter, the CPU 201a stores the obtained circulating blood volume ($A_x$) in the nonvolatile memory 201b at a step S406. Thus, the subroutine of data analysis at the step S111 in Fig. 4 ends, and the process returns to a master routine (processing at the step S113 and subsequent steps).

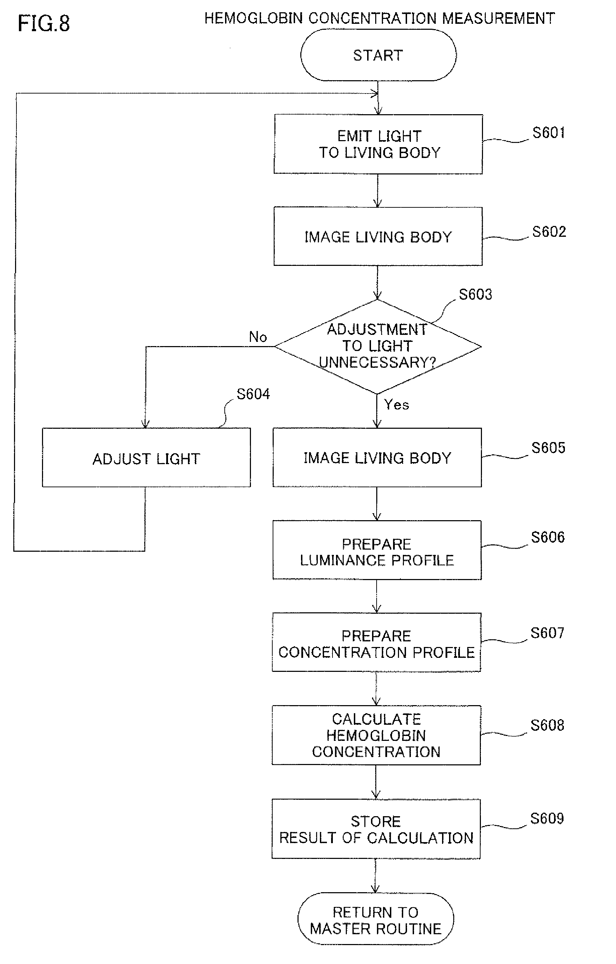

Next, a subroutine of Hb concentration measurement by the detecting portion 3 at the step S203 in Fig. 4 is described with reference to Fig. 8.

First, the CPU 301a executes processing for emitting light to a living body by the light source portion 34 at a step S601 in Fig. 8. Next, the CPU 301a executes processing for imaging the living body to which the light is emitted by the imaging portion 35 at a step S602. At a step S603, the CPU 301a determines whether or not adjustment to light of the light source portion 34 is unnecessary on the basis of luminance of the image obtained at the aforementioned step S602. More specifically, the CPU 301a determines that the adjustment to light is unnecessary when the luminance of the obtained image is higher than a prescribed threshold and determines that the adjustment to light is necessary when the luminance of the obtained image is lower than the prescribed threshold. When determining that the adjustment to light is necessary, the CPU 301a moves the process to a step S604, and the process is returned to the step S601 after the CPU 301a executes processing (adjustment to light) for increasing current supplied to the light source portion 34 through the light source portion input/output interface 301d.

When determining that the adjustment to light is unnecessary, on the other hand, the CPU 301a advances the process to a step S605 and executes processing for imaging the living body by the imaging portion 35 again.

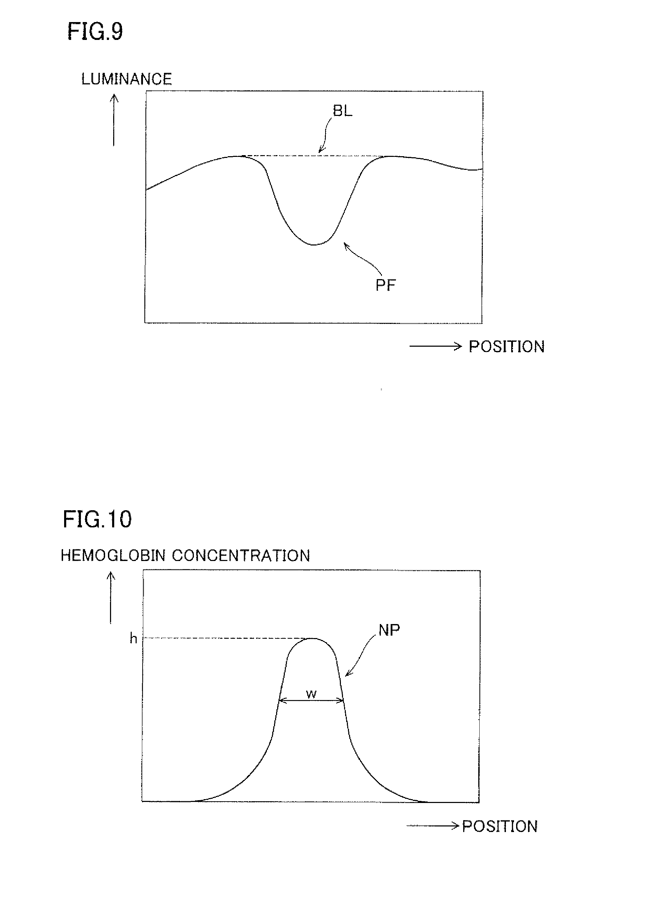

Next, at a step S606, the CPU 301a executes processing for preparing a luminance profile PF shown in Fig. 9 on the basis of the image obtained by the imaging portion 35. Near-infrared light highly absorbed by hemoglobin is emitted to the wrist, and the wrist to which the near-infrared light is emitted is imaged, whereby an image in which a blood vessel in the wrist darkly appears is obtained. In other words, the near-infrared light is absorbed by hemoglobin contained in blood flowing in the blood vessel, whereby luminance of a region corresponding to the blood vessel lowers as compared with luminance of a region other than the blood vessel in the imaged image. A luminance distribution distributed across this blood vessel image is extracted, whereby the luminance profile PF reflecting a concentration of hemoglobin contained in the blood vessel of the wrist is obtained.

Next, at a step S607, the CPU 301a executes processing for preparing a concentration profile NP reflecting the concentration of hemoglobin contained in the blood vessel shown in Fig. 10. More specifically, as shown in Fig. 9, the CPU 301a obtains a baseline BL from the luminance profile PF and standardizes the luminance profile PF on the basis of this baseline BL, thereby preparing the concentration profile NP (see Fig. 10). The baseline BL is a straight line obtained from a luminance profile PF of a portion other than the blood vessel by a least-squares method and corresponds to luminance of a background of the blood vessel. The luminance profile PF is standardized on the basis of the baseline BL, whereby the concentration profile NP independent from an incident light intensity can be obtained, as shown in Fig. 10.

Next, at a step S608, the CPU 301a acquires a peak height h (a maximum value of Hb concentration), which is a morphological feature, and a distribution width w of the concentration profile NP at a height h/2 from the concentration profile NP and executes processing for calculating an Hb concentration C on the basis of the following formula (4):

$$C = h/w^n \quad ... \quad (4)$$

In the aforementioned formula (4), n is a constant number indicating a nonlinear of a half-value width caused by light scattering. When there is no light scattering, n = 1, and when there is light scattering, n > 1.

Thereafter, at a step S609, the CPU 301a executes processing for storing the obtained Hb concentration (Hb concentration C) in the main memory 301b, and the process returns to a master routine (processing at the step S204 and subsequent steps in Fig. 4).

According to this embodiment, as hereinabove described, the CPU 301a of the detecting portion 3 analyzes the living body image obtained by the imaging portion 35 to acquire the Hb concentration, and the CPU 201a of the PC 2 acquires the circulating blood volume and outputs the circulating blood volume graph showing the fluctuation of the obtained circulating blood volume by the display portion 202, whereby the changes of the circulating blood volume can be noninvasively monitored.

According to this embodiment, as hereinabove described, the circulating blood volume is acquired on the basis of the initial value ($C_0$) of the Hb concentration and the latest Hb concentration ($C_x$), whereby the circulating blood volume ($A_x$) can be acquired on the basis of Hb concentrations at two points which are the initial value ($C_0$) of the Hb concentration and the latest Hb concentration ($C_x$) (at the certain point x).

According to this embodiment, as hereinabove described, the circulating blood volume ($A_x$) at the certain point x is acquired on the basis of the initial value ($C_0$) of the Hb concentration, the latest Hb concentration ($C_x$) and the initial circulating blood volume ($A_0$), whereby the circulating blood volume ($A_x$) can be acquired on the basis of the circulating blood volume (initial circulating blood volume ($A_0$)) normally contained in the living body.

According to this embodiment, as hereinabove described, the circulating blood volume ($A_x$) at the certain point x is acquired by accumulating the blood dilution rate ($D_x$) which is a change rate of hemoglobin concentration at the certain point x and the initial circulating blood volume ($A_0$), and the obtained circulating blood volume ($A_x$) is output to the display portion 202. According to this structure, a circulating blood volume corresponding to the change rate of the Hb concentration can be acquired, and hence the user can see a change of the circulating blood volume at a glance by outputting the circulating blood volume ($A_x$) corresponding to the change of the Hb concentration and the initial circulating blood volume ($A_0$).

According to this embodiment, as hereinabove described, the circulating blood volume graph showing the fluctuation of the circulating blood volume over time is generated on the basis of the initial circulating blood volume ($A_0$) and the latest blood dilation rate ($D_x$) and displayed on the display portion 202, whereby information (a graph) with which the user easily visually understands the change of the circulating blood volume can be output.

Experimental Example

In order to confirm the effects of the monitoring apparatus 1 according to this embodiment, the following experiment was performed.

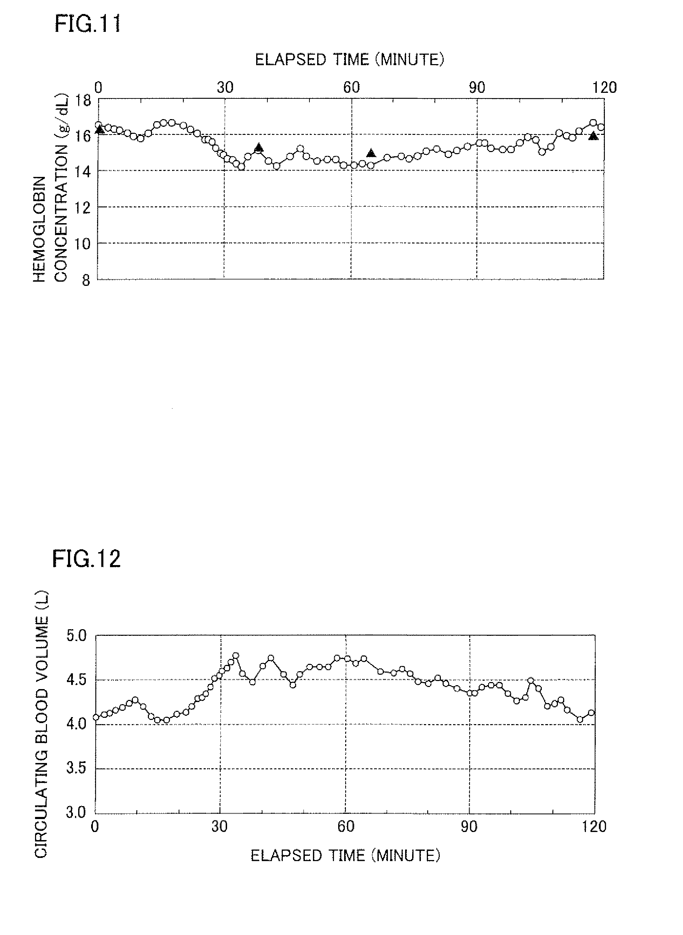

A change of Hb concentration was monitored for 120 minutes with the monitoring apparatus 1 according to this embodiment by having a healthy adult male take 660 mL of hypotonic electrolyte fluid orally. Blood was collected four times in total in parallel with the monitoring, and respective actual measured values of Hb concentration were obtained. Fig. 11 shows the results. In Fig. 11, results of measurement of Hb concentration obtained by the monitoring apparatus 1 according to this embodiment are denoted by circles, and actual measured values of Hb concentration obtained by collecting the blood are denoted by triangles.

As shown in Fig. 11, Hb concentrations obtained by the monitoring apparatus 1 according to this embodiment, and Hb concentrations obtained by collecting the blood did not differ from each other, and it has been verified that an accurate Hb concentration approximate to an actual Hb concentration can be acquired by the monitoring apparatus 1 according to this embodiment.

Further, as evidenced by Fig. 11, it has been observed that the Hb concentrations decrease over time after oral intake of fluid, and thereafter the Hb concentrations gradually increase. An extremely minute amount of blood is collected, and hence decreases of the Hb concentrations observed between 30 minutes and 60 minutes after oral intake can be presumed to result from dilution of circulating blood by intake of fluid. Increases of the Hb concentrations observed between 60 minutes and 120 minutes after oral intake of fluid can be presumed to result from transport of unnecessary fluid ingested into the circulating blood to the urinary organs and recovery of Hb concentration in the blood to original state. Thus, it has been verified that the circulating blood is diluted by oral intake of fluid and changes of the Hb concentration are produced. This suggested that the degree of absorption of fluid orally taken can be estimated by monitoring Hb concentration.

Next, a correlation between the blood dilution rate and the degree of fluid absorption was considered.

First, the circulating blood volume of the subject was obtained from the height and weight of the subject. In this experiment, the height of the subject is 174.5 cm, and the weight is 54.6 kg. When calculating the circulating blood volume on the basis of the aforementioned formula (1), the circulating blood volume of the subject was calculated to be 4.07 L. Since the amount of fluid taken by the subject is 660 mL, the circulating blood volume of the subject is 4.73 L and the rate of increase in the circulating blood volume is 1.16 times, assuming that all the taken fluid was absorbed in the living body.

Next, the blood dilution rate in the subject was obtained. The Hb concentration at the start of measurement obtained by the monitoring apparatus 1 according to this embodiment was 16.6 g/dL, and the Hb concentration at the lowest point was 14.1 g/dL. When calculating the blood dilution rate on the basis of this ratio, the blood dilution rate in the subject was calculated to be 117 %. This value is substantially equi to the rate of increase in the circulating blood volume calculated above. Therefore, it has been verified that the blood dilution rate obtained by the monitoring apparatus according to this embodiment is a value accurately reflecting the degree of fluid absorption.

Fig. 12 is a graph showing changes of the circulating blood volume on the basis of the results shown in Fig. 11. As shown in Fig. 12, it can be observed that the circulating blood volume increases over time after oral intake of fluid and gradually decreases. This graph is obtained on the basis of the blood dilution rate, and it can be said that the graph shown in Fig. 12 accurately reflects the change of the circulating blood volume caused by fluid absorption.

Thus, it has been verified that the degree of fluid absorption can be accurately estimated by monitoring the blood dilution rate. Further, it has been verified that the change of the circulating blood volume caused by fluid absorption can be monitored by monitoring the circulating blood volume.

The embodiment disclosed this time must be considered as illustrative in all points and not restrictive. The range of the present invention is shown not by the above description of the embodiment but by the scope of claims for patent, and all modifications within the meaning and range equivalent to the scope of claims for patent are included.

For example, while the graph showing the relation between time lapse and the circulating blood volume is output as information about the changes of the circulating blood volume in this embodiment, the present invention is not restricted to this structure. For example, the initial circulating blood volume and the current circulating blood volume may be displayed. Alternatively, the initial circulating blood volume may be set to 100 %, and the rate of increase in the circulating blood volume may be displayed as a percentage. Alternatively, how much the circulating blood volume has been recovered with respect to the initial circulating blood volume may be displayed, if the circulating blood volume of the subject is reduced by collecting the blood, as compared with the initial circulating blood volume.

While the blood dilution rate and the circulating blood volume are calculated and the graphs showing those fluctuations are displayed in the monitoring apparatus 1 according to this embodiment, the present invention is not restricted to this structure. For example, only the graph showing the fluctuation of the circulating blood volume may be displayed.

While the PC 2 for performing data analysis and the detecting portion 3 measuring an Hb concentration are formed separately in the monitoring apparatus 1 according to this embodiment, the present invention is not restricted to this structure. For example, the application program implemented by the monitoring apparatus 1 according to this embodiment may be run by the detecting portion 3.

While the structure of performing measurement of Hb concentration in the detecting portion 3 has been shown in the monitoring apparatus 1 according to this embodiment, the present invention is not restricted to this structure. For example, the detecting portion 3 may acquire an image, and the PC 2 may analyze the obtained image to acquire an Hb concentration.

While the example of storing data such as the results of measurement of hemoglobin concentration and the calculated circulating blood volume in the nonvolatile memory 201b has been shown in this embodiment, the present invention is not restricted to this. In the present invention, the data such as the results of measurement of Hb concentration and the calculated circulating blood volume may be stored in the RAM 201c or the hard disk 201d other than the nonvolatile memory 201b, for example.

While the example of measuring a hemoglobin concentration (Hb concentration) to measure the changes of the circulating blood volume of the subject has been shown in this embodiment, the present invention is not restricted to this. In the present invention, a blood component concentration other than a hemoglobin concentration may be measured to measure the changes of the circulating blood volume of the subject.

Industrial Applicability

The monitoring apparatus according to this embodiment can be utilized as follows: For example, the monitoring apparatus is placed in a location for performing collection of blood such as a blood donation center, a subject from whom blood has been collected orally takes fluid, and thereafter the circulating blood volume of the subject is monitored by the monitoring apparatus. If it can be confirmed that the circulating blood volume has been sufficiently recovered, it can be determined that the risk of a VVR is small, and if it cannot be confirmed that, another action such as continuous fluid intake can be taken. Thus, in the monitoring apparatus according to this embodiment, useful action to inhibit the risk of a VVR can be taken without taking invasive action.

**Claims**

1.  A living body monitoring apparatus, comprising:

    image acquisition means emitting light to a living body and acquiring a living body image by imaging the living body to which the light is being emitted;
    concentration acquisition means acquiring a concentration of a blood component of said living body by analyzing said living body image obtained by said image acquisition means;
    information, acquisition means acquiring information about a change of a circulating blood volume contained in said living body on the basis of said concentration of said blood component obtained by said concentration acquisition means; and
    output means outputting said information about a change of a circulating blood volume obtained by said information acquisition means.

2.  The living body monitoring apparatus according to claim 1, wherein
    said information acquisition means is configured to acquire said information about a change of a circulating blood volume on the basis of a first concentration of said blood component obtained by analyzing a first living body image obtained by said image acquisition means and a second concentration of said blood component obtained by analyzing a second living body image obtained after acquisition of said first living body image.

3.  The living body monitoring apparatus according to claim 2, further comprising standard circulating blood volume acquisition means acquiring a standard circulating blood volume capable of being calculated from a height and a weight of said living body, wherein
    said information acquisition means acquires said information about a change of a circulating blood volume on the basis of said first concentration of said blood component, said second concentration of said blood component and said standard circulating blood volume.

4.  The living body monitoring apparatus according to claim 3, wherein
    said information acquisition means acquires a circulating blood volume in said living body at the time of acquisition of said second living body image by obtaining a change rate of said concentration of said blood component on the basis of said first concentration of said blood component and said second concentration of said blood component and accumulating said change rate of said concentration of said blood component and said standard circulating blood volume, and
    said output means outputs said standard circulating blood volume and said circulating blood volume in said living body at the time of acquisition of said second riving body image as said information about a change of a circulating blood volume.

5.  The living body monitoring apparatus according to any one of claims 1 to 4, wherein
    said blood component is hemoglobin.

6.  The living body monitoring apparatus according to any one of claims 2 to 4, wherein
    said information acquisition means is configured to be capable of acquiring said information about a change of a circulating blood volume on the basis of said first concentration of said blood component obtained by analyzing said first living body image, said second concentration of said blood component obtained by analyzing said second living body image, and a third concentration of said blood component obtained by analyzing a third living body image obtained after acquisition of said second living body image.

7. The living body monitoring apparatus according to any one of claims 1 to 6, wherein
said output means is configured to output a graph showing a relation between time lapse and said circulating blood volume contained in said living body as said information about a change of a circulating blood volume.

8. The living body monitoring apparatus according to claim 3 or 4, further comprising input acceptance means accepting input of body information about a height and a weight of a living body, wherein
said standard circulating blood volume acquisition means acquires said standard circulating blood volume on the basis of said body information accepted by said input acceptance means.

9. The living body monitoring apparatus according to claim 8, wherein
said input acceptance means is further configured to be capable of accepting input of a volume of collected blood collected from said living body, and
said standard circulating blood volume acquisition means is configured to be capable of acquiring said standard circulating blood volume on the basis of said body information and said volume of collected blood accepted by said input acceptance means.

10. A living body monitoring apparatus, comprising:

    an image acquisition portion emitting light to a living body and acquiring a living body image by imaging the living body to which the light is being emitted;
    an output portion; and
    a control portion acquiring a concentration of a blood component of said living body by analyzing said living body image obtained by imaging said living body by said image acquisition portion, acquiring information about a change of a circulating blood volume contained in said living body on the basis of obtained said concentration of said blood component, and outputting obtained said information about a change of a circulating blood volume to said output portion.

11. The living body monitoring apparatus according to claim 10, wherein
said control portion is configured to:

    acquire a first concentration of said blood component by analyzing a first living body image obtained by said image acquisition portion,
    acquire a second concentration of said blood component by analyzing a second living body image obtained after acquisition of said first living body image, and
    acquire said information about a change of a circulating blood volume on the basis of said first concentration of said blood component and said second concentration of said blood component.

12. The living body monitoring apparatus according to claim 11, wherein
said control portion is configured to:

    acquire a standard circulating blood volume on the basis of information of a height and a weight of said living body, and
    acquire said information about a change of a circulating blood volume on the basis of said first concentration of said blood component, said second concentration of said blood component and said standard circulating blood volume.

13. The living body monitoring apparatus according to claim 12, wherein
said control portion is configured to:

    obtain a change rate of said concentration of said blood component on the basis of said first concentration of said blood component and said second concentration of said blood component,
    acquire a circulating blood volume in said living body at the time of acquisition of said second living body image from a product of said change rate and said standard circulating blood volume, and
    output said standard circulating blood volume and said circulating blood volume in said living body at the time of acquisition of said second living body image to said output portion as said information about a change of a circulating blood volume.

**14.** The living body monitoring apparatus according to any one of claims 10 to 13, wherein said blood component is hemoglobin.

**15.** The living body monitoring apparatus according to claim 11 or 12, wherein said control portion is configured to acquire said information about a change of a circulating blood volume on the basis of said first concentration of said blood component obtained by analyzing said first living body image, said second concentration of said blood component obtained by analyzing said second living body image, and a third concentration of said blood component obtained by analyzing a third living body image obtained after acquisition of said second living body image.

**16.** The living body monitoring apparatus according to any one of claims 10 to 15, wherein said control portion is configured to output a graph showing a relation between time lapse and said circulating blood volume contained in said living body to said output portion as said information about a change of a circulating blood volume.

**17.** The living body monitoring apparatus according to claim 12, wherein said control portion is configured to:

accept input of body information about a height and a weight of said living body, and acquire said standard circulating blood volume on the basis of accepted said body information.

**18.** The living body monitoring apparatus according to claim 17, wherein said control portion is configured to:

accept input of a volume of collected blood collected from said living body, and acquire said standard circulating blood volume on the basis of accepted said body information and said volume of collected blood.

**19.** The living body monitoring apparatus according to any one of claims 10 to 17, wherein said control portion includes a first control portion acquiring said concentration of said blood component of said living body by analyzing said living body image obtained by imaging said living body by said image acquisition portion, and a second control portion acquiring said information about a change of a circulating blood volume contained in said living body on the basis of said concentration of said blood component obtained by said first control portion and outputting obtained said information about a change of a circulating blood volume to said output portion.

FIG.1

# FIG.2

**FIG.3**

DETECTING PORTION
3
301

CONTROL PORTION

301a — CPU

301g

301b — MAIN MEMORY → INPUT INTERFACE ← OPERATING PORTION — 32

301e — FRAME MEMORY ↔ IMAGE OUTPUT INTERFACE → DISPLAY PORTION — 33
301i

301j — FLASH MEMORY CARD

301c — FLASH MEMORY CARD READER ↔ LIGHT SOURCE PORTION INPUT/OUTPUT INTERFACE ← LIGHT SOURCE PORTION — 34
301d

CA

TO PC 2 ← INPUT/OUTPUT INTERFACE ↔ IMAGE INPUT INTERFACE ← IMAGING PORTION — 35
301f

301h

FLOW OF PROCESSING
BY DETECTING PORTION 3

FLOW OF PROCESSING
BY PC 2

FIG.4

# FIG.5

BLOOD DILUTION RATE (%)

402a

| | | |
|---|---|---|
| 110 | | |
| 105 | | |
| 100 | | |
| 95 | | |

0    20    40    60    80    100    120 MIN.

CIRCULATING BLOOD VOLUME (L)

| | |
|---|---|
| 4.4 | |
| 4.2 | |
| 4.0 | |
| 3.8 | |

0    20    40    60    80    100    120 MIN.

402b

402

STANDBY                401

403

ID :                          403a

HEIGHT :               cm      403b

WEIGHT :               kg      403c

AMOUNT OF
FLUID INTAKE :          mL      403d

COLLECTION
OF BLOOD :              dL      403e

40

MEASUREMENT START          404

MEASUREMENT END          405

EP 2 347 708 A1

## FIG.6

**IN PROCESS OF MEASUREMENT** (501)

**BLOOD DILUTION RATE (%)** (502a)

```
110
105
100
 95
     0   20   40   60   80  100  120 MIN.
```

**CIRCULATING BLOOD VOLUME (L)** (502b)

```
4.4
4.2
4.0
3.8
     0   20   40   60   80  100  120 MIN.
```

| ID: | 000 |
| HEIGHT: | 175cm |
| WEIGHT: | 62.5kg |

| AMOUNT OF FLUID INTAKE: | 200mL |
| COLLECTION OF BLOOD: | 200dL |

MEASUREMENT START (504)

MEASUREMENT END (505)

EP 2 347 708 A1

FIG.7

DATA ANALYSIS

START

READ INITIAL
Hb CONCENTRATION (C$_0$) AND
LATEST Hb CONCENTRATION (C$_x$) — S401

CALCULATE
BLOOD DILUTION RATE (D$_x$) — S402

STORE
BLOOD DILUTION RATE (D$_x$) — S403

READ INITIAL CIRCULATING
BLOOD VOLUME (A$_0$) — S404

CALCULATE CIRCULATING
BLOOD VOLUME (A$_x$) — S405

STORE CIRCULATING
BLOOD VOLUME (A$_x$) — S406

RETURN TO
MASTER ROUTINE

FIG.8

HEMOGLOBIN CONCENTRATION MEASUREMENT

START

EMIT LIGHT
TO LIVING BODY — S601

IMAGE LIVING BODY — S602

ADJUSTMENT
TO LIGHT
UNNECESSARY? — S603

No

Yes

ADJUST LIGHT — S604

IMAGE LIVING BODY — S605

PREPARE
LUMINANCE PROFILE — S606

PREPARE
CONCENTRATION PROFILE — S607

CALCULATE
HEMOGLOBIN
CONCENTRATION — S608

STORE
RESULT OF CALCULATION — S609

RETURN TO
MASTER ROUTINE

FIG.9

LUMINANCE

BL

PF

POSITION

FIG.10

HEMOGLOBIN CONCENTRATION

NP

h

w

POSITION

## FIG.11

ELAPSED TIME (MINUTE)

HEMOGLOBIN CONCENTRATION (g/dL)

## FIG.12

CIRCULATING BLOOD VOLUME (L)

ELAPSED TIME (MINUTE)

# INTERNATIONAL SEARCH REPORT

| | International application No. |
|---|---|
| | PCT/JP2009/067873 |

A. CLASSIFICATION OF SUBJECT MATTER
*A61B5/1455*(2006.01)i, *A61B5/026*(2006.01)i

According to International Patent Classification (IPC) or to both national classification and IPC

B. FIELDS SEARCHED

Minimum documentation searched (classification system followed by classification symbols)
A61B5/1455, A61B5/026

Documentation searched other than minimum documentation to the extent that such documents are included in the fields searched
| Jitsuyo Shinan Koho | 1922–1996 | Jitsuyo Shinan Toroku Koho | 1996–2009 |
| Kokai Jitsuyo Shinan Koho | 1971–2009 | Toroku Jitsuyo Shinan Koho | 1994–2009 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y<br>A | Toshiyuki OZAWA·Kaoru ASANO·Shigehiro NUMADA·Yasushi HASUI·Yasuhiro KOCHI·Ken ISHIHARA, "Kinsekigai Bunko Gazo Keisokuho ni yoru Kecchu Hemoglobin Nodo no Mushinshu Sokutei", Transactions of Japanese Society for Medical and Biological Engineering, 10 March 2005 (10.03. 2005), vol.43, no.1, pages 93 to 101 | 1-2,10-11<br>3-9,12-19 |
| Y | JP 63-275326 A (Hamamatsu Photonics Kabushiki Kaisha), 14 November 1988 (14.11.1988), page 4, upper left column to upper right column (Family: none) | 1-2,10-11 |

☒ Further documents are listed in the continuation of Box C.  ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 13 November, 2009 (13.11.09) | 24 November, 2009 (24.11.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| International application No. |
| --- |
| PCT/JP2009/067873 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 6-277202 A (Hamamatsu Photonics Kabushiki Kaisha), 04 October 1994 (04.10.1994), paragraphs [0012], [0024] & US 5564418 A | 1-2,10-11 |

Form PCT/ISA/210 (continuation of second sheet) (April 2007)

**EP 2 347 708 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001502590 A